# EUROPEAN PATENT SPECIFICATION

(11) **EP 1 937 168 B1**
(45) Date of publication and mention of the grant of the patent: **24.02.2010**
(21) Application number: 06808344.3
(22) Date of filing: 20.10.2006
(51) Int. Cl.: A61B 17/70

(54) **A SPINAL SUPPORT ROD KIT**
WIRBELSÄULENSTÜTZSTANGEN-KIT
ENSEMBLE TIGE DE MAINTIEN VERTEBRAL

(30) Priority: 22.10.2005 GB 0521585
(43) Date of publication of application: 02.07.2008
(73) Proprietor: Depuy International Limited, Beeston, Leeds LS11 8DT (GB)
(72) Inventor: FIRKINS, Paul, 2000 Neuchatel (CH); SANDERS, Marc, 7553 AM Hengelo (NL); SMITH, Richard, Memphis, TN 38112 (US)
(74) Representative: Belcher, Simon James
(86) International application number: PCT/GB2006/003912
(87) International publication number: WO 2007/045895

(56) References cited:
- EP-A1- 0 470 660
- EP-A2- 1 364 622
- WO-A-02/080788
- DE-A1-102004 010 844
- US-A- 5 282 863

## Description

The present invention relates to a spinal support rod kit for the treatment of spinal column shape deformations.

A spinal support rod can be used in the treatment of spinal column shape deformations. For example, spinal support rods have been used for many years in the treatment of scoliosis. In particular, spinal support rods can be used to treat lateral curvature and/or axial turning of the spinal column. To treat deformations, the spinal support rod is attached to the vertebrae along at least part of the length of the spinal column by an attachment assembly.

It is known to form spinal support rods from shape memory alloy materials, for example as disclosed in EP-A-470660. A rod can be made from a shape memory alloys in a configuration which is stable while the alloy is in its austenite phase, cooled so that the alloy exists in its martensite phase, deformed to a configuration which matches that of the deformed spinal column, attached to the patient's vertebrae, and then heated to a temperature at which the phase of the alloy changes from martensite to austenite. The shape of the rod then tends towards the initial stable configuration, imposing corrective forces on the spine to which it is attached. The viscoelastic qualities of the spinal column allow deformation of the spinal column to be corrected.

It is generally desirable to restrict relative movement between the spinal support rod and the attachment assembly. In particular, it can be important to restrict the rod rotation of the rod within the attachment assembly. It can also be desirable to restrict axial movement of the spinal support rod relative to the attachment assembly. It can further be desirable to allow easy detachment of the spinal support rod from the attachment assembly to allow repositioning of the spinal support rod.

WO-02/080788 discloses a spinal osteo-synthesis device which comprises at least one rod having two flat sides and a rounded side, and an anchor for the rod. The rod can be inserted into a channel in the anchor. A clamping screw acts against one of the flat sides of the rod, and presses the opposite flat side against a flat clamping surface within the anchor.

The present invention provides a spinal support rod kit which includes a spinal support rod having a round face and a flat face.

Accordingly, in one aspect, the invention provides a spinal support rod kit as defined in claim 1.

The provision of a flat side on the rod for flush contact with a flat sides of the channel of the retaining portions helps to restrict the rotation of the rod within the attachment assembly. This is advantageous over known round spinal support rods which can tend to rotate within their attachment assembly.

Further, it has been found that engagement between the rounded side of the rod and the rounded side of the channel can provide sufficient resistance to slippage of the rod through the attachment assembly, while still allowing the spinal support rod to be easily detached from the attachment assembly. This can be in contrast to square rods which prevent slippage of the rod through the attachment assembly by relying on an interference fit between the spinal support rod and the attachment assembly. Such interference fits can be difficult to release which can cause problems when the surgeon needs to revise the position of the spinal support rod.

The provision of a round side to the spinal support rod provides the advantage that the spinal support rod can be less likely to become jammed in the channel of the attachment assembly compared with support rods with a square cross-section.

The control over the engagement of the rod of the present invention in the attachment assembly has the further advantage that it can facilitate precise and accurate location of the support rod in the attachment assembly because of the greater ease with which the rod can be located in the assembly, and then released and relocated as required.

Accordingly, it has been found that the provision of a spinal support rod having a flat side and a rounded side, and an attachment assembly comprising retaining portions each of which defines a channel for receiving the rod in which the cross-sectional shape of the channel includes a rounded side for tightly engaging the rounded side of the rod, can be particularly advantageous as it provides good resistance to axial rotation of the rod within the attachment assembly, allows for easy repositioning of the spinal support rod, while still providing sufficient resistance to axial slippage of the spinal support rod.

The rounded side of the support rod can be regular or irregular in shape when the rod is viewed in cross-section. For example, the rounded side of the support rod can have the approximate shape of a part of an ellipse when the rod is viewed in cross-section. Preferably, the rounded side of the support rod has the approximate shape of an arc of a circle when the rod is viewed in cross-section.

Preferably, the angle subtended by the arc can be selected according to the nature of the engagement that is required between the rod and the channel. Engagement around a greater angle of arc can be preferred in order to provide good resistance to rotation around the axis of the rod, and resistance to axial movement. For example the angle subtended by the arc might be at least about 45 °, preferably at least about 60 °, possibly at least about 90 °, more preferably at least about 120°, especially at least about 150°. It can be particularly preferred that the arc subtends an angle of at least about 170°, for example, about 180° (i.e. the rounded side of the support rod has the approximate shape of a semi-circle when the rod is viewed in cross-section). Smaller angles of arc can be suitable for some applications.

The rounded side of the channel can be regular or irregular in shape. For example, the rounded side of the channel can have the approximate shape of a part of an ellipse when the rod is viewed in cross-section. Preferably, the rounded side of the channel has the approximate shape of an arc of a circle when the channel is viewed from one side.

Preferably, the rounded side of the channel has the approximate shape of an arc of a circle when the channel is viewed from one side. Preferably, the rod is a snug fit in the channel, more preferably, an interference fit so that play between the rod and the channel is minimised, at least when the rod is tightened into the channel. It can be particularly preferred that the radius of the rounded side of the channel is not more than the radius of the rounded side of the support rod. This can help to ensure that the rounded side of the channel tightly engages the rounded side of the support rod to restrict movement of the rod through the channel in a direction generally parallel to the axis of the rod.

It can be preferable that the radius of the rounded side of the channel and the radius of the rounded side of the support rod are such that the channel engages the rod sufficiently to restrict movement of the rod through the channel in a direction generally parallel to the axis of the rod while allowing the surgeon to easily disengage the spinal support rod from the retaining portion of the attachment assembly. Preferably, the rod is larger than the width of the channel so that the internal walls of the channel grip the rod when it is inserted into the channel. Preferably, the difference between the radius of the rounded side of the rod and the radius of the rounded side of the channel is at least about 50 µm, for example about 75 µm. When the rod is larger than the channel in which it is intended to fit, it can be preferred to use an internally threaded circumferential nut which engages a thread on the external wall of the channel in order to retain the rod in the channel. Such a nut can support the walls of the channel against being splayed by the action of the rod on the internal walls of the channel.

When an externally threaded screw is located within the channel, engaging threads on the internal walls of the channel, to retain the support rod in the channel, it will generally preferable for the support rod to be slightly smaller than the width of the channel so that it is a sliding fit within the channel.

Preferably, the cross-sectional shape of the spinal support rod includes second and third flat sides, together with the said first flat side and the said rounded side. Preferably, the cross-sectional shape of the channel also includes second and third flat sides for flush contact with the two flat sides of the spinal support rod, together with the first flat side and the said rounded side. Accordingly, all three flat sides can both provide resistance to the rotation of the spinal support rod within the retaining portion.

Preferably, the rod which includes three flat sides is arranged so that two of the flat sides are extend approximately parallel to one another, with the rounded side and the third flat side located opposite to one another, along opposite edges of the parallel sides.

It has been found that great resistance to the rotation of the spinal support rod within the retaining assembly can be provided when the first flat side of the support rod is adjacent to the rounded side.

It can be advantageous to provide for significant resistance to rotation of the spinal support rod within the attachment assembly at the ends of the spinal support rod. Preferably, the side of the support rod that is rounded along at least part of its length is flat at at least one end of the rod. For example, preferably the cross-sectional shape of the support rod towards at at least one end of the rod is approximately square. Preferably, the rounded side of the channel of the retaining portion of the attachment assembly that secures one of the ends of the spinal support rod to the spinal column is flat. For example, preferably the cross-sectional shape of the channels of the retaining portions of the attachment assembly that secure the ends of the spinal support rod to the spinal column is approximately square. Preferably, the rounded sides of the channels of the retaining portions of the attachment assembly that secures the spinal support rod to the spinal column towards the ends of the spinal support rod, are flat.

As discussed in the paragraph below, it can be advantageous to allow for some movement of the spinal support rod within the channel of a retaining portion at points in-between the ends of the spinal support rod, and particularly towards the middle of the spinal support rod. Accordingly, in some circumstances it can be preferable for the cross-sectional shape of the spinal support rod to be rounded towards its middle so that the force by which the spinal support rod is held against rotation and sliding within the channel of a retaining portion towards the middle of the spinal support rod is less than that at the ends of the spinal support rod.

Preferably, the kit includes first and second retaining portions for securing first and second ends of the spinal support rod to the spinal column at spaced apart points along the spinal column. Preferably, the kit includes a third retaining portion for securing the spinal support rod to the spinal column at a point between the first and second ends of the support rod. In some circumstances it can be preferable that the forces by which the rod is held against rotation and sliding within the channels of the first and second retaining portions is greater than the forces by which the rod is held against rotation and sliding within the channel of the third retaining portion. In this way, the support rod will be restrained against movement relative to attachment assemblies at its ends, but limited movement can be permitted at points along its length. Such limited movement can be desirable for some patients. It can be achieved by restricting the tension that can be applied to a spinal support rod within a fixation channel. For example, when the rod is retained within a channel by means of a threaded fastener such as a screw or a nut or both, one of the threads on the channel and the fastener can include a discontinuity to restrict relative rotation between the channel and the fastener.

The cross-sectional shape of the spinal support rod can be approximately constant along at least part, especially the central part, of its length.

The cross-sectional area of the support rod can be approximately constant along its length. It can be advantageous for the cross-sectional area of the spinal support rod to be greater at some points along its length than at other points. It can be preferable that the rod cross-sectional area of the spinal support rod is greater at points which will be subject to greater forces. Preferably, the cross-sectional area of the support rod is greater at at least one of its ends than at its other end. More preferably, the cross-sectional area of the spinal support rod is greater at its end that will be proximal the lumbar area of the spinal column.

The shape and size of the spinal support rod and the channel of the retaining portion can be configured so that the spinal support rod can be secured within the channel by way of an interference fit between the spinal support rod and the retaining device.

The channel can be open so that the spinal support rod can be slid into the channel in a direction generally transverse to the axis of the spinal support rod.

The kit includes a clamping device for clamping the spinal support rod within the channel. Examples of suitable clamping devices include those which are used in existing spinal support assemblies. For example, the Moss Miami system which is sold by DePuy Spine Inc includes circumferentially threaded screws which can engage a thread on the internal wall of a rod-receiving channel, and includes internally threaded nuts which can engage a thread on the external wall of a rod-receiving channel. Such screws or such nuts or both can be used in the kit of the present invention.

Preferably, the clamping device can be separated from and subsequently reattached to the channel. This can be advantageous as the clamping device can be removed to allow for the spinal support rod to be slid into and out of the channel in a direction generally transverse to the axis of the spinal support rod.

The clamping device can provide a side of the channel. The clamping device can provide additional flat sides of the channel.

A wall of the channel is threaded. The thread can be provide on an external face of the channel. The thread can be provided on an internal wall of the channel. Preferably, the clamping device comprises a screw which can engage the thread on the channel, so that it can be tightened to clamp the spinal support rod between the screw and the rounded side of the channel.

The anchor portion can be any type of mechanism by which the retaining portion can be attached to a patient's vertebrae. Preferred mechanisms include those disclosed in EP-A-470660 and US-5391168.

The spinal support rod will generally be formed from a metal. Suitable materials can include titanium and its alloys, and certain stainless steels. Preferably, the spinal support rod is formed from a shape memory alloy. For example, the spinal support rod can be made from nickel titanium based shape memory alloy. Other materials for the rod might include titanium and its alloys. Fittings for use with the support rod, such as connectors between rod segments and devices for fixing the rod to a patient's vertebrae can be made from titanium and its alloys, or from stainless steel. The selection of materials for spinal support rods and for fittings for use therewith can be in accordance with existing products from DePuy Spine Inc and from other companies.

The spinal support rod can be made using conventional techniques for making rods, with appropriate modification for making the spinal support rod according to the invention. Techniques including milling, grinding, and hot and cold working techniques such as drawing. The rod can initially be made as a round rod using conventional techniques and then modified to include a flat side by techniques such as milling or grinding.

In another aspect, the invention provides a spinal support rod for use in the treatment of ' spinal column shape deformations, in which the cross-sectional shape of the spinal support rod taken perpendicular to its length, comprises a first flat side and a rounded side.

Embodiments of the invention will now be described, by way of example only, and with reference to the accompanying drawings, in which:
Figure 1 is an end view of a spinal support rod received within a retaining portion of a spinal support rod kit according to the invention; and
Figure 2 is a perspective view of the spinal support rod shown in Figure 1; and
Figure 3 is a cross-sectional view of the spinal support rod shown in Figure 1, taken perpendicular to its longitudinal axis.
Figure 4 is an isometric view of a spinal support rod and fixation screws which can be used to fix it to a patient's vertebrae.

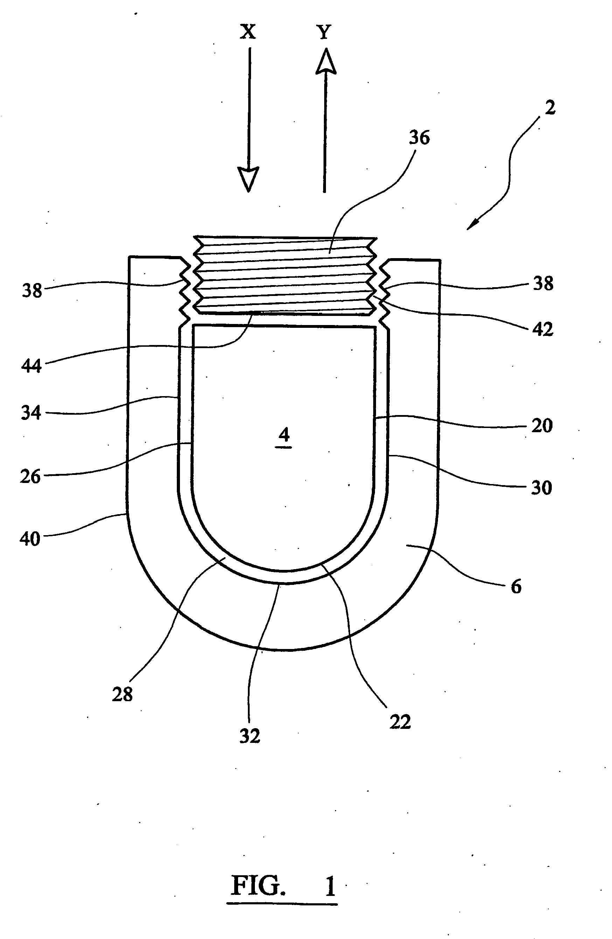

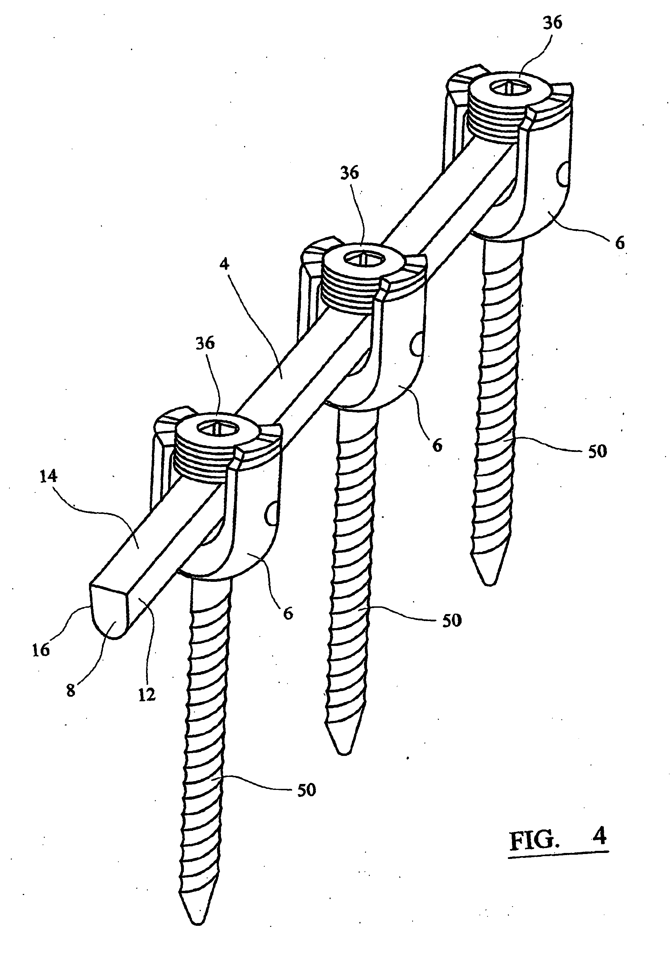

Referring to the drawings, Figure 1 shows a spinal support rod kit 2 which comprises a spinal support rod 4 and a retaining portion 6 of an attachment assembly. The spinal support rod kit 2 is shown in isolation for simplicity and ease of illustration. However, it will be appreciated that the retaining portion 6 will be attached to a vertebra by an anchor portion (not shown). The anchor portion can be any suitable mechanism for securing the retaining portion 6 to a vertebra, such as a pedicle screw, or a hook. It will also be appreciated that the attachment assembly of the spinal support rod kit will comprise a plurality of retaining portions 6. Typically, at least two retaining portions 6 will be provided for securing the spinal support rod 4 at two points to the spinal column, across the part of the spinal column that is to be treated. Figure 4 shows the spinal support rod 4 received within three retaining portions 6. In this embodiment, the spinal support rod 4 will be secured to a patient's spinal column at three points. As illustrated, the retaining portions have fixation screws 50 for fixing the retaining portions to a patient's vertebrae (not shown).

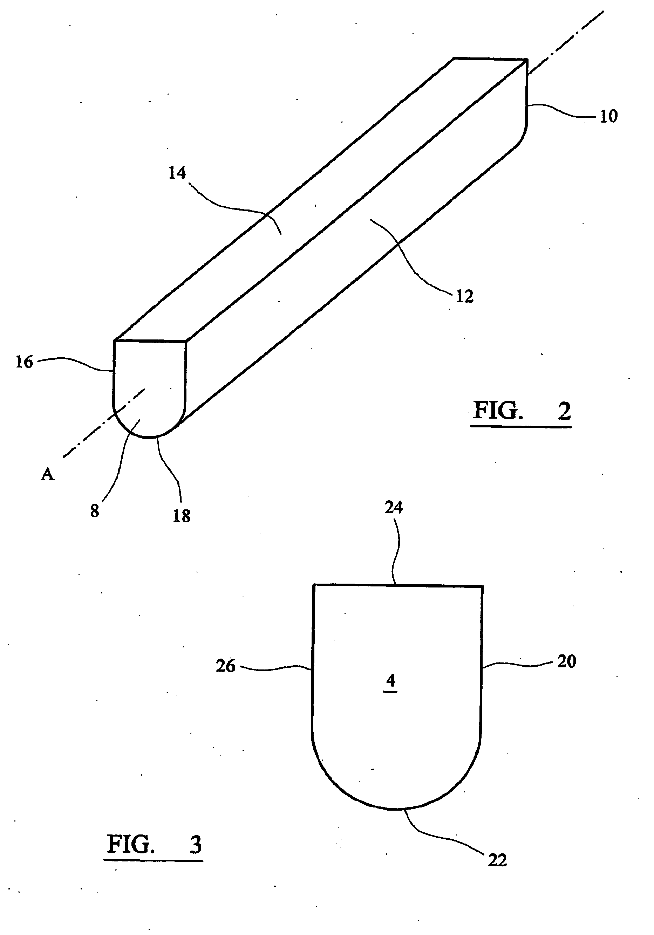

With reference to Figure 2, the spinal support rod 4 has first 8 and second 10 ends. First 12, second 14 and third 16 flat walls and a rounded wall 18 extend between the first 8 and second 10 ends of the spinal support rod 4.

As shown in Figure 3, the cross-sectional shape of the spinal support rod 4, taken in a plane perpendicular to its longitudinal axis A, comprises a first flat side 20 defined by the first flat wall 12, and a rounded side 22 defined by the rounded wall 18. The cross-sectional shape further includes second 24 and third 26 flat sides defined by second 14 and third 16 flat walls. The cross-sectional shape and size of the spinal support rod 4 is approximately constant along its entire length between the first 8 and second 10 ends.

Referring back to Figure 1, the wall 40 of the retaining portion 6 is approximately U-shaped when viewed from one side. The retaining portion 6 of the attachment assembly defines a channel 28 for receiving the spinal support rod 4. The cross-sectional shape of the channel 28 includes first 30 and second 34 flat sides for flush contact with the first 20 and second 26 flat side 20 of the spinal support rod 4, and a rounded side 32, extending between the first 30 and second flat 34 sides, which engages the rounded side 22 of the spinal support rod when the spinal support rod is fully received within the retaining portion 6. The spinal support rod 4 and the retaining portion 6 are shaped and sized so that the spinal support rod is a snug fit within the channel 28.

The wall 40 of the retaining portion 6 is threaded at the ends 38 of the first 30 and second 34 flat sides distal to the rounded side 32, for threaded engagement with a clamping device 36 (described in more detail below).

The retaining device 6 comprises a clamping device 36. In the embodiment shown, the clamping device 36 is a set screw having a threaded wall 42 for threaded engagement with the threaded on the wall 40 of the retaining portion 6. The set screw has a first end 46 for engagement with a tool (not shown) to rotate the set screw, and a second end 44. When the set screw is received within the walls 40 of the retaining portion, the second end 44 provides a third flat side of the channel 28 for flush contact with the second flat side 14 of the spinal support rod 4.

In the embodiment shown, the radius of the rounded side 22 of the spinal support rod 4 is approximately 3.0 mm, and the radius of the rounded side 32 of the retaining portion 6 is approximately 3.05 mm Accordingly, the difference between the radii is approximately 0.05 mm. The spinal support rod is therefore a sliding fit in the channel which is defined within the retaining portion. Locating the rod within the channel does not require outward deformation of the walls of the channel.

In use, the retaining portion 6 is secured to a spinal column via an anchor portion (not shown). The clamping device 36 is initially removed from the walls 40 of the retaining portion 6 and the spinal support rod 4 is slid into the channel 28 in a direction perpendicular to the axis of the spinal support rod, as illustrated by arrow X.

When the spinal support rod 4 is fully received within the retaining portion 6, the first 20 and third 26 flat sides of the spinal support rod fit flush against the first 30 and second 34 flat sides of the channel 28, thereby restricting rotation of the spinal support rod within the retaining portion 6.

Also, the difference in the radii of the rounded 22 side of the spinal support rod 4 and the rounded side 32 of the retaining portion 6 is such that when the spinal support rod is fully received in the retaining portion, the rounded side of the retaining portion tightly engages the rounded side of the spinal support rod. This engagement restricts sliding of the spinal support rod 4 through the retaining portion in a direction approximately parallel to its longitudinal axis.

Once the spinal support rod 4 is fully received within the retaining portion 6, the set screw 36 is fastened to the walls 40 of the retaining portion and is tightened so that the spinal support rod 4 is clamped between the third flat side of the channel 28 provided by the flat end 44 of the set screw and the rounded side 32 of the retaining portion.

When the spinal support rod 4 is properly clamped in the retaining portion 6, the spinal support rod is further restricted from sliding through the retaining portion in a direction approximately parallel to its longitudinal axis.

To remove the spinal support rod 4 from the retaining portion 6, the set screw 36 is loosened and removed from the walls 40 of the retaining portion. The spinal support rod 4 is then pulled out of the retaining portion 6 in a direction perpendicular to its longitudinal axis as illustrated by arrow Y.

## Claims

1. A spinal support rod kit for the treatment of spinal column shape deformations, comprising:
(a) a spinal support rod (4) having first and second ends (8, 10), in which the cross-sectional shape of the rod along at least part of its length, taken perpendicular to its longitudinal axis, includes a first flat side (12), a second flat side (16) opposite the first flat side, and a rounded side (18) between the first and second flat sides such that the first and second flat sides extend from respective edges of the rounded side, and
(b) an attachment assembly for securing the support rod to the spinal column at at least two points along its length, comprising retaining portions (6) each of which defines a channel (28) for receiving the rod and an anchor portion (50) for attachment to a patient's vertebrae, in which the cross-sectional shape of the channel (28) when viewed from one side includes a first flat side (30), a second flat side (34) opposite the first flat side, and a rounded side (32) between the first and second flat sides such that the first and second flat sides extend from respective edges of the rounded side, a wall of the channel being threaded,
(c) a clamping device (36) which is provided by a threaded screw or a threaded nut which can threadingly engage the thread on the wall of the channel,
**characterised in that** the rod can be positioned in the channel with the first and second flat sides of the channel in flush contact with the first and second flat sides respectively of the rod, and with the rounded side of the channel clamped against the rounded side of the rod by the action of the clamping device so as to restrict movement of the rod through the channel in a direction generally parallel to the axis of the rod.

2. A spinal support rod kit as claimed in claim 1, in which the rounded side (18) of the support rod (4) has the approximate shape of an arc of a circle when the rod is viewed in cross-section.

3. A spinal support rod kit as claimed in claim 2, in which the arc subtends an angle of at least about 90°.

4. A spinal support rod kit as claimed in claim 1, in which the rounded side (32) of the channel (28) has the approximate shape of an arc of a circle when the channel is viewed from one side.

5. A spinal support rod kit as claimed in claim 2, in which the rounded side (32) of the channel (28) has the approximate shape of an arc of a circle when the channel is viewed from one side, and in which the radius of the rounded side of the channel is not more than the radius of the rounded side (18) of the rod (4).

6. A spinal support rod kit as claimed in claim 1, in which the cross-sectional shape of the support rod (4) includes at least one further flat side (14).

7. A spinal support rod kit as claimed in claim 1, in which the side (18) of the support rod (4) that is rounded along at least part of its length is flat at at least one end of the rod.

8. A spinal support rod kit as claimed in claim 7, in which the cross-sectional shape of the support rod (4) towards the said end is approximately square.

9. A spinal support rod kit as claimed in claim 1, in which the cross-sectional area of the support rod (4) is greater at one of its ends than at its other end.

10. A spinal support rod kit as claimed in claim 1, in which the clamping device (36) provides a third flat side of the channel.

11. A spinal support rod kit as claimed in claim 1, in which the rod (4) is formed from a shape memory alloy.

## Patentansprüche

1. Basisausrüstung mit einer Stange zum Stützen von Wirbeln bei der Behandlung von Formdeformationen der Wirbelsäule, die aufweist:
(a) eine Stange (4) zum Stützen von Wirbeln mit einem ersten und einem zweiten Ende (8, 10), wobei die Querschnittsform der Stange über wenigstens einen Teil ihrer Länge, senkrecht zu ihrer Längsachse, eine erste flache Seite (12), eine zweite flache Seite (16) gegenüber der ersten flachen Seite und eine gerundete Seite (18) zwischen der ersten und der zweiten flachen Seite umfasst, so dass sich die erste und die flache Seite von jeweiligen Kanten der gerundeten Seite her erstrecken, und
(b) eine Befestigungsanordnung zum Sichern der stützenden Stange an der Wirbelsäule an wenigstens zwei Punkten über ihre Länge, die Haltebereiche (6) aufweist, von denen jeder einen Kanal (28) zum Aufnehmen der Stange und einen Ankerbereich (50) zum Befestigen an einem Wirbel des Patienten definiert, wobei die Querschnittsform des Kanals (28), wenn von einer Seite betrachtet wird, eine erste flache Seite (30), eine zweite flache Seite (34) gegenüber der ersten flachen Seite und eine gerundete Seite (32) zwischen der ersten und der zweiten flachen Seite umfasst, so dass sich die erste und die zweite flache Seite von jeweiligen Kanten der gerundeten Seite erstrecken, wobei eine Wand des Kanals mit Gewinde versehen ist,
(c) eine Klemmeinheit (36), die durch eine mit Gewinde versehende Schraube oder eine mit Gewinde versehene Mutter gebildet ist, die in das Gewinde der Wand des Kanals eingreifen kann,
**dadurch gekennzeichnet, dass** die Stange in dem Kanal mit der ersten und der zweiten flachen Seite des Kanals in bündigem Kontakt mit der ersten bzw. der zweiten flachen Seite der Stange positioniert werden kann und die gerundete Seite des Kanals gegen die gerundete Seite der Stange durch die Wirkung der Klemmeinheit verklemmt ist, um so die Bewegung der Stange durch den Kanal in eine Richtung im Allgemeinen parallel zu der Achse der Stange zu beschränken.

2. Basisausrüstung mit einer Stange zum Stützen von Wirbeln nach Anspruch 1, bei der die gerundete Seite (18) der stützenden Stange (4) die ungefähre Form eines Kreisbogens hat, wenn die Stange im Querschnitt betrachtet wird.

3. Basisausrüstung mit einer Stange zum Stützen von Wirbeln nach Anspruch 2, bei der der Bogen einem Winkel von wenigstens ungefähr 90° gegenübersteht.

4. Basisausrüstung mit einer Stange zum Stützen von Wirbeln nach Anspruch 1, bei der die gerundete Seite (32) des Kanals (28) die ungefähre Form eines Kreisbogens, wenn der Kanal von einer Seite her betrachtet wird, hat.

5. Basisausrüstung mit einer Stange zum Stützen von Wirbeln nach Anspruch 2, bei der die gerundete Seite (32) des Kanals (28) die ungefähre Form eines Kreisbogens, wenn der Kanal von einer Seite her betrachtet wird, hat, und bei der der Radius der gerundeten Seite des Kanals nicht größer ist als der Radius der gerundeten Seite (18) der Stange (4).

6. Basisausrüstung mit einer Stange zum Stützen von Wirbeln nach Anspruch 1, bei der die Querschnittsform der stützenden Stange (4) wenigstens eine weitere flache Seite (14) umfasst.

7. Basisausrüstung mit einer Stange zum Stützen von Wirbeln nach Anspruch 1, bei der die Seite (18) der stützenden Stange (4), die über wenigstens einen Teil ihrer Länge gerundet ist, an wenigstens einem Ende der Stange flach ist.

8. Basisausrüstung mit einer Stange zum Stützen von Wirbeln nach Anspruch 7, bei der die Querschnittsform der stützenden Stange (4) auf das Ende zu ungefähr quadratisch ist.

9. Basisausrüstung mit einer Stange zum Stützen von Wirbeln nach Anspruch 1, bei der die Querschnittsfläche der stützenden Stange (4) an einem ihrer Enden größer ist als an ihrem anderen Ende.

10. Basisausrüstung mit einer Stange zum Stützen von Wirbeln nach Anspruch 1, bei der die Klemmeinheit (36) eine dritte flache Seite des Kanals bildet.

11. Basisausrüstung mit einer Stange zum Stützen von Wirbeln nach Anspruch 1, bei der die Stange (4) aus einer Legierung mit Formgedächtnis gebildet ist.

## Revendications

1. Ensemble tige de maintien de la colonne vertébrale destiné à traiter des déformations de la forme de la colonne vertébrale, comprenant :
(a) une tige de maintien (4) de la colonne vertébrale qui présente des première et deuxième extrémités (8, 10), dans lequel la forme en coupe transversale de la tige le long d'une partie au moins de sa longueur, prise perpendiculaire à son axe longitudinal, comprend un premier côté plat (12), un deuxième côté plat (16) opposé au premier côté plat, et un côté arrondi (18) situé entre les premier et deuxième côtés plats de telle sorte que les premier et deuxième côtés plats s'étendent àpartir des bords respectifs du côté arrondi,et
(b) un ensemble fixation destiné à fixer la tige de maintien sur la colonne vertébrale au niveau de deux points au moins sur sa longueur, comprenant des parties de retenue (6), chacune d'elles définissant un canal (28) destiné à recevoir la tige et une partie d'ancre (50) pour une fixation sur les vertèbres d'un patient, dans lequel la forme en coupe transversale du canal (28), vue à partir d'un côté, comprend un premier côté plat (30), un deuxième côté plat (34) opposé au premier côté plat, et un côté arrondi (32) situé entre les premier et deuxième côtés plats de telle sorte que les premier et deuxième côtés plats s'étendent à partir des bords respectifs du côté arrondi, une paroi du canal étant filetée,
(c) un dispositif de serrage (36) qui est fourni par une vis filetée ou un écrou fileté qui peut venir en prise par vissage avec le filetage situé sur la paroi du canal,
**caractérisé en ce que** la tige peut être positionnée dans le canal avec les premier et deuxième côtés plats du canal en contact affleurant avec les premier et deuxième côtés plats respectivement de la tige, et le côté arrondi du canal étant serré contre le côté arrondi de la tige grâce à l'action du dispositif de serrage de manière à limiter le déplacement de la tige à travers le canal dans une direction en général parallèle à l'axe de la tige.

2. Ensemble tige de maintien de la colonne vertébrale selon la revendication 1, dans lequel le côté arrondi (18) de la tige de maintien (4) présente une forme approximative en arc de cercle lorsque la tige est vue en section transversale.

3. Ensemble tige de maintien de la colonne vertébrale selon la revendication 2, dans lequel l'arc sous-tend un angle au moins égal à 90° environ.

4. Ensemble tige de maintien de la colonne vertébrale selon la revendication 1, dans lequel le côté arrondi (32) du canal (28) présente une forme approximative en arc de cercle lorsque le canal est vu à partir d'un côté.

5. Ensemble tige de maintien de la colonne vertébrale selon la revendication 2, dans lequel le côté arrondi (32) du canal (28) présente une forme approximative en arc de cercle lorsque le canal est vu à partir d'un côté, et dans lequel le rayon du côté arrondi du canal n'est pas supérieur au rayon du côté arrondi (18) de la tige (4).

6. Ensemble tige de maintien de la colonne vertébrale selon la revendication 1, dans laquelle la forme en coupe transversale de la tige de maintien (4) comprend au moins un autre côté plat (14).

7. Ensemble tige de maintien de la colonne vertébrale selon la revendication 1, dans lequel le côté (18) de la tige de maintien (4) qui est arrondie le long d'une partie au moins de sa longueur est plat au moins au niveau d'une extrémité de la tige.

8. Ensemble tige de maintien de la colonne vertébrale selon la revendication 7, dans laquelle la forme en coupe transversale de la tige de maintien (4) vers ladite extrémité est approximativement carrée.

9. Ensemble tige de maintien de la colonne vertébrale selon la revendication 1, dans laquelle la section de la coupe transversale de la tige de maintien (4) est plus grande au niveau de l'une de ses extrémités qu'au niveau de l'autre extrémité.

10. Ensemble tige de maintien de la colonne vertébrale selon la revendication 1, dans lequel le dispositif de serrage (36) fournit un troisième côté plat du canal.

11. Ensemble tige de maintien de la colonne vertébrale selon la revendication 1, dans lequel la tige (4) est réalisée dans un alliage à mémoire de forme.
